# EUROPÄISCHE PATENTSCHRIFT

(11) **EP 3 007 825 B1**
(45) Veröffentlichungstag und Bekanntmachung des Hinweises auf die Patenterteilung: **06.10.2021**
(21) Anmeldenummer: 14731184.9
(22) Anmeldetag: 06.06.2014
(51) Int. Cl.: B01L 3/02, A61M 5/34

(54) **SPRITZE MIT MECHANISCHEM KOPPLUNGSSTÜCK**
SYRINGE HAVING A MECHANICAL COUPLING PIECE
SERINGUE POURVUE D'UNE PIÈCE DE COUPLAGE MÉCANIQUE

(30) Priorität: 13.06.2013 DE 102013210998
(43) Veröffentlichungstag der Anmeldung: 20.04.2016
(73) Patentinhaber: Hamilton Bonaduz AG, 7402 Bonaduz (CH)
(72) Erfinder: LECKEBUSCH, Klaus, CH-7425 Masein (CH)
(74) Vertreter: Müller Hoffmann & Partner
(86) Internationale Anmeldenummer: PCT/EP2014/061912
(87) Internationale Veröffentlichungsnummer: WO 2014/198673

(56) Entgegenhaltungen:
- GB-A- 2 202 148
- US-A- 2 764 978
- US-A- 4 281 653
- US-A- 4 720 285
- US-A1- 2009 163 859
- None

## Beschreibung

Die vorliegende Erfindung betrifft eine Spritze, insbesondere eine Spritze für eine Dosiervorrichtung zur Verwendung bei Chormatographieanwendungen, wie etwa Hochtemperaturchromatographie oder Headspace-Chromatographie.

Eine derartige Spritze umfasst einen Spritzenkörper mit einer axialen Innenausnehmung, die sich in Richtung einer Längsachse des Spritzenkörpers erstreckt und in der ein Kolben in Längsrichtung verschiebbar aufnehmbar oder aufgenommen ist, und eine Nadel, die an einem Längsende des Spritzenkörpers mit diesem verbindbar oder verbunden ist, wobei der Spritzenkörper an seinem nadelseitigen Längsende einen Kopplungsabschnitt aufweist, an dem die Nadel mittels eines in Längsrichtung aufschiebbaren Verbindungselements befestigbar oder befestigt ist.

Eine solche Spritze zur Verwendung im Laborbereich ist beispielsweise aus der WO 2009/036994 A2 bekannt. Bei der bekannten Spritze sind die Nadel und der Spritzenkörper mittels eines hülsenförmigen Führungsbauteils miteinander verklebt, wobei das Führungsbauteil ein Innengewinde aufweist, in das eine mit der Nadel verbundene Spannschraube eingeschraubt ist. Aus der US 4 720 285 A ist eine Injektionsspritze bekannt, bei der ein Nadelträger mittels radial vorstehender Klauen mit dem Spritzenkörper verbunden wird.

Ebenfalls US2764978 A, GB2202148 A, US2009/163859 A1, sowie US4281653 A offenbaren verschiedene Arten von Kopplungen zwischen Nadel und Spritze. Ferner sind auch aus der Medizin verschiedene Kopplungen von Nadeln und Spritzenkörpern bekannt, die allerdings für Anwendungen im Laborbereich nicht ausreichend sind. Sie erfüllen insbesondere nicht die Anforderungen an Festigkeit und vor allem Dichtigkeit. Dabei entsteht das Problem, dass die Reinheit eines von der Spritze aufzunehmenden oder abzugebenden Fluids nicht im gewünschten Maß sichergestellt werden kann.

Es hat sich gezeigt, dass Klebeverbindungen bei Spritzen für Dosiervorrichtungen aus dem Laborbereich nachteilig sind, da bei ihrem Einsatz in Analysegeräten, wie etwa (Gas-) Chromatographie eine Kontamination der Probe in der Spritze mit insbesondere flüchtigen organischen Stoffen aus dem Klebstoff aufgetreten sind. Diese flüchtigen Stoffe gasen bei Erwärmung der Spritze aus dem Klebstoff aus und können die mit dem Analysegerät erhaltenen Messergebnisse über das Probenfluid signifikant beeinflussen. Ferner ist es auch schwierig, Spritzen mit Klebeverbindungen in Umgebungen mit Lösungsmitteln, z.B. als Probenfluid zu verwenden, da die verwendeten Klebstoffe durch die Lösungsmittel geschädigt werden können.

Es ist daher Aufgabe der Erfindung, eine Spritze für eine Dosiervorrichtung bereitzustellen, bei der diese Nachteile vermieden werden können.

Zur Lösung dieser Aufgabe wird eine Spritze mit den Merkmalen des Anspruchs 1 vorgeschlagen.

Bei einer eingangs erwähnten Spritze ist also am Kopplungsabschnitt wenigstens eine radial nach innen ausgebildete Ausnehmung vorgesehen, die mit wenigstens einem korrespondierenden am Verbindungselement vorgesehenen Rastabschnitt in Eingriff bringbar ist oder steht.

Die Ausnehmung am Kopplungsabschnitt und der Rastabschnitt am Verbindungselement ermöglichen dabei eine rein mechanische Verbindung zwischen Nadel und Spritzenkörper, bei der die Verwendung von Klebstoffen vermieden werden kann. Das Einrasten des Rastabschnitts in die Ausnehmung erfolgt dabei in einfacher Weise beim axialen Aufschieben des Verbindungselements auf den Kopplungsabschnitt, so dass auch die Herstellung einer solchen Spritze vereinfacht ist, verglichen mit einer Herstellung einer Spritze mit Klebeverbindung und ggf. mit einer Spannschraubenkopplung zwischen Nadel und Verbindungselement.

Weiter ist vorgesehen, dass das Verbindungselement als Hülse ausgebildet ist, die einen spritzen - körperseitigen Verbindungsabschnitt und einen nadelseitigen Stützabschnitt umfasst, wobei der wenigstens eine Rastabschnitt am Verbindungsabschnitt angeordnet ist.

Weiter ist vorgesehen, dass am Verbindungsabschnitt mehrere in Umfangsrichtung verteilte Rastabschnitte vorgesehen sind, die sich entlang des Innenumfangs der Hülse erstrecken und radial nach innen weisen.

Weiter ist vorgesehen, dass der Verbindungsabschnitt im Bereich der Rastabschnitte in Längsrichtung verlaufende Einschnitte aufweist, die am spritzenkörperseitigen Ende der Hülse beginnen und in Richtung zum nadelseitigen Ende der Hülse weisen, derart, dass die Rastabschnitte mehrere Rastabschnittsteile aufweisen, die an entsprechenden Verbindungsabschnittsteilen angeordnet sind, wobei die Verbindungsabschnittsteile in radialer Richtung auslenkbar sind.

Durch eine derartige Konstruktion kann der Rasteingriff zwischen Verbindungselement und Kopplungsabschnitt an mehreren entlang des Umfangs verteilten Stellen erfolgen, so dass eine sichere formschlüssige Verbindung gewährleistet werden kann. Mittels der (elastisch) auslenkbaren Verbindungsabschnittsteile wird das axiale Aufschieben des Verbindungselements auf den Kopplungsabschnitt ermöglicht, auch wenn die radial nach innen weisenden Rastabschnitte bzw. Rastabschnittteile den Innendurchmesser des Verbindungsstücks im Bereich der Rastabschnitte verkleinern. Die Verbindungsabschnittsteile liegen beim axialen Aufschieben auf den Kopplungsabschnitt mit den Rastabschnittsteilen am Kopplungsabschnitt an, wobei die Verbindungsabschnittsteile in radialer Richtung nach außen verformt bzw. gebogen bis die die Rastabschnittsteile bzw Rastabschnitte in die Ausnehmung am Kopplungsabschnitt eingreifen. Dabei Bewegen sich die Verbindungsabschnittsteile beim Einrasten aufgrund der elastischen Verformung schnappend nach radial innen.

Um die Dichtigkeit der Verbindung zwischen Spritzenkörper und Nadel zu erhöhen, ist zusätzlich vorgesehen, dass die Spritze eine Spanneinrichtung aufweist, mittels welcher eine axiale Vorspannung zwischen Kopplungsabschnitt und Verbindungselement herstellbar oder hergestellt ist, die auf Dichtungselemente wirkt, welche zwischen dem Kopplungsabschnitt und dem Verbindungselement aufgenommen sind.

Dabei ist vorgesehen, dass die Spanneinrichtung die wenigstens eine Ausnehmung am Kopplungsabschnitt umfasst, wobei sich die Ausnehmung entlang des Umfangs des Kopplungsabschnitts erstreckt und in Form eines Gewindegangs ausgebildet ist.

Dabei ist es bevorzugt, dass der Gewindegang derart ausgebildet ist, dass er in beide Drehrichtungen einen Übergang zu einer Außenumfangsfläche des Kopplungsabschnitts aufweist, und dass vorzugsweise maximal eine Umdrehung des Verbindungselements um den Kopplungsabschnitt möglich ist, vorzugsweise eine Teildrehung um etwa 1/8 bis 7/8 des Umfangs des Kopplungsabschnitts.

Der vorgeschlagene Gewindegang ist kein herkömmliches Gewinde, bei dem die Verbindung von zwei korrespondierenden Bauteilen durch eine reine relative Drehbeweung erreicht wird. Vielmehr dient der Gewindegang, der durch die Ausnehmung am Kopplungsabschnitt der Spritze gebildet wird, lediglich einer zusätzlichen axialen Verspannung nach Herstellen des Rasteingriffs zwischen den Rastabschnitten und der Ausnehmung. Der Eingriff zwischen den Rastabschnitten und der Ausnehmung erfolgt durch die beschriebene Schnappverbindung bereits beim axialen Aufschieben des Verbindungselements auf den Kopplungsabschnitt ohne dass hierzu eine anfängliche Verdrehung der beiden Teile zueinander erforderlich ist wie bei einem herkömmlichen Gewinde. Diese Kombination aus dem axialem Aufschieben mit Einrasten der Rastabschnitte in die Ausnehmung und aus dem anschließenden Verdrehen des Verbindungsabschnitts um etwa 1/8 bis 7/8 des Umfangs des Kopplungabschnitts vereinfacht die Herstellung solcher Spritzen und ermöglicht eine sicher und hochdichte mechanische form- und reibschlüssige Verbindung zwischen der Nadel und dem Spritzenkörper.

Es wird ferner vorgeschlagen, dass die Hülse an ihrem Stützabschnitt eine radial nach innen weisende Begrenzungswandung aufweist, durch deren Zentrum die Nadel geführt ist, wobei im zusammengesetzten Zustand der Spritze die Begrenzungswandung mit ihrer dem Spritzenkörper zugewandten Innenfläche eine axiale Kraft auf die an einer axialen Stirnfläche des Kopplungsabschnitts anliegenden Dichtungselemente ausübt.

Hierzu ist weiterbildend vorgesehen, dass der Stützabschnitt einen radial nach innen weisenden Stabilisierungsring aufweist, der im zusammengesetzten Zustand der Spritze an der Stirnfläche des Kopplungsabschnitts anliegt, wobei vorzugsweise der Stabilisierungsring einstückig mit der Hülse ausgebildet ist.

Die Dichtungselemente und die Nadel sind vorzugsweise im Verbindungselement bzw. in der Hülse aufgenommen, vorzugsweise sind sie darin befestigt, so dass sie als gemeinsames Bauteil mit dem Kopplungsabschnitt des Spritzenkörpers verbindbar oder verbunden sind.

Ferner wird vorgeschlagen, dass der Spritzenkörper und der Kopplungsabschnitt einstückig ausgebildet sind, vorzugsweise aus Glas.

Das Verbindungselement bzw. die Hülse können aus einem Kunststoff gebildet sein.

Um eine präzise Aufnahme der Nadel im Verbindungsabschnitt zu ermöglichen, wird vorgeschlagen, dass die Spritze eine im Verbindungselement bzw. der Hülse aufgenommene Nadelhalterung umfasst, welche ein spritzenkörperseitiges Ende einer Kanüle der Nadel in Umfangsrichtung dicht umschließt und im zusammengesetzten Zustand der Spritze mittels Dichtungselementen zwischen dem Verbindungselement bzw. der Hülse und der nadelseitigen Stirnfläche des Kopplungsabschnitts gehalten ist.

Nachfolgend wird die Erfindung unter Bezugnahme auf die anliegenden Figuren beispielhaft und nicht einschränkend beschrieben.
- Fig. 1: zeigt eine vereinfachte und schematische Längsschnittdarstellung einer Ausführungsform einer Spritze mit Spritzenkörper und Nadel.
- Fig. 2: zeigt eine Vergrößerung des in Fig. 1 mit II bezeichneten Verbindungsbereichs zwischen Spritzenkörper und Nadel mit Verbindungselement und Kopplungsabschnitt.
- Fig. 3: zeigt eine Vergrößerung des Verbindungselements und dessen Rastabschnitte entsprechend dem Bereich III der Fig. 2 in zwei Alternativen.
- Fig. 4: zeigt in Teilfigur a) eine schematische und vereinfachte perspektivische Darstellung des Spritzenkörpers und in Teilfigur b) eine Vergrößerung des mit IV bezeichneten Bereichs der Fig. 3a).
- Fig. 5: zeigt eine vereinfachte und schematische Perspektivdarstellung eines Verbindungselements, das die Kopplung zwischen Nadel und Spritzenkörper ermöglicht.
- Fig. 6: zeigt vereinfacht und schematisch in einer Schnittdarstellung den Ablauf des axialen Aufschiebens des Verbindungselements auf den Kopplungsabschnitt und das Herstellen einer Rastverbindung zwischen diesen Bauteilen (Schritte a-d).

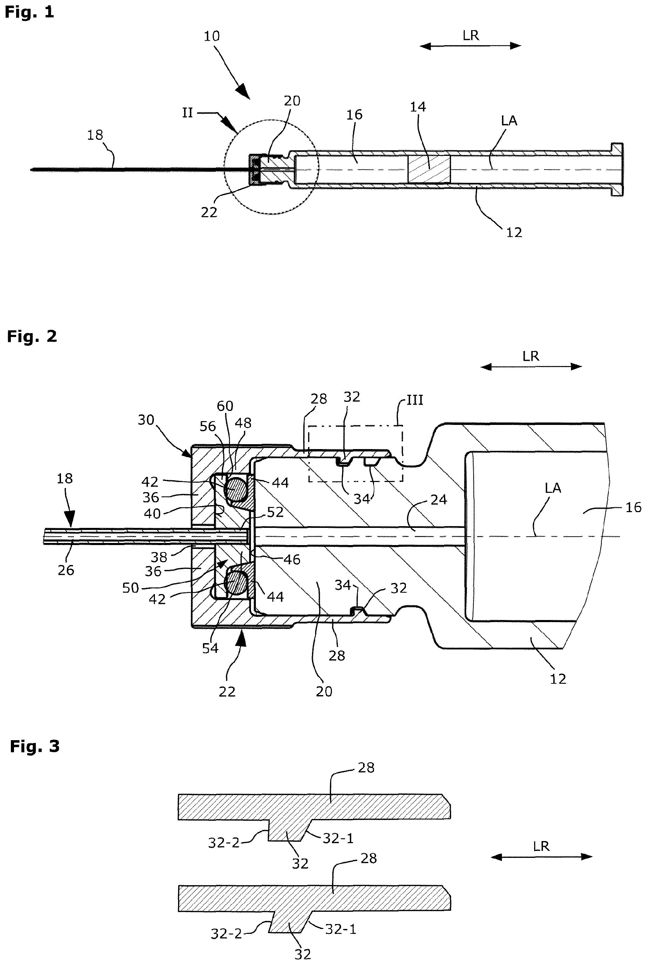

In Fig. 1 ist eine Ausführungsform einer Spritze 10 vereinfacht und schematisch in einem Längsschnitt entlang der Längsachse LA dargestellt. Die Spritze 10 umfasst einen Spritzenkörper 12, in dem ein Kolben 14 entlang der Längsrichtung LR beweglich aufgenommen ist. Der Kolben 14 ist mit einer hier nicht dargestellten Betätigungseinrichtung verbunden, so dass das Volumen eines Aufnahmeraums 16 (Innenausnehmung des Spritzenkörpers 12) für ein Fluid, das mittels der Spritze 10 aufgenommen oder abgegeben werden soll, verändert werden kann. Mit dem Spritzenkörper 12 ist eine Nadel 18 verbunden. Die Kopplung zwischen der Nadel 18 und dem Spritzenkörper 12 mittels eines am Spritzenkörper 12 vorgesehenen Kopplungsabschnitts 20 und eines nadelseitig vorgesehenen Verbindungselements 22, das vorzugsweise als eine Art Hülse ausgebildet ist, die in axialer Längsrichtung LR auf den Kopplungsabschnitt 20 aufgeschoben werden kann.

Die Verbindung zwischen Nadel 18 und Spritzenkörper 12 wird nun anhand der vergrößerten schematischen Schnittsdarstellung der Fig. 2 erläutert, die im Wesentlichen dem in Fig. 1 mit II bezeichneten kreisförmigen Bereich entspricht.

Der Kopplungsabschnitt 20 ist vorzugsweise einstückig mit dem Spritzenkörper 12 verbunden. Der Aufnahmeraum 16 steht mittels eines im Kopplungsabschnitt 20 ausgebildeten Kanals 24 mit einer Kanüle 26 der Nadel 18 in Fluidverbindung, wenn das Verbindungselement 22 und die Nadel 18 mit dem Kopplungsabschnitt 20 verbunden sind.

Das Verbindungselement 22, das nachfolgend auch als Hülse 22 bezeichnet wird, umfasst einen spritzenkörperseitigen Verbindungsabschnitt 28 und einen nadelseitigen Stützabschnitt 30. Am Verbindungsabschnitt 28 ist wenigstens ein Rastabschnitt 32 vorgesehen, der radial nach innen zur Längsachse LA gerichtet ist bzw. vorsteht. Der bzw. die Rastabschnitt(e) 32 stehen im zusammengesetzten Zustand der Spritze 10 mit einer am Kopplungsabschnitt 20 ausgebildeten Ausnehmung 34 in Eingriff. Die Ausnehmung 34 und der bzw. die Rastabschnitt(e) 32 weisen korrespondierende Profile auf, so dass durch das Ineinadergreifen des bzw. der Rastabschnitt(e) 32 und der Ausnehmung 34 eine formschlüssige mechanische Verbindung zwischen dem Verbindungselement 22 und dem Kopplungsabschnitt 20 hergestellt ist bzw. hergestellt werden kann.

An seinem Stützabschnitt 30 weist das Verbindungselement 22 eine radial nach innen weisende Begrenzungswandung 36 auf, in deren Zentrum eine Öffnung 38 vorgesehen ist, durch welche die Nadel 18 geführt ist. Im zusammengesetzten Zustand der Spritze 10 übt die Begrenzungswandung 36 mit ihrer dem Spritzenkörper zugewandten Innenfläche 40 eine axiale Kraft auf Dichtungselemente 42, 44 aus, so dass diese gegen eine axiale Stirnfläche 46 des Kopplungsabschnitts 20 gepresst bzw. vorgespannt werden. Im vorliegenden Ausführungsbeispiel ist zwischen der Begrenzungswandung 36 und den Dichtungselementen 42, 44 in axialer Richtung ferner ein Der Stützabschnitt weist ferner einen radial nach innen weisenden Stabilisierungsringabschnitt 48 auf, der im zusammengesetzten Zustand der Spritze 10 der Stirnseite 46 des Kopplungsabschnitts 20 gegenüber liegt. Der Stabilisierungsring 48 ist dabei bevorzugt einstückig mit der Hülse 22 ausgebildet.

In der Hülse 22 ist ferner eine Nadelhalterung 50 ersichtlich, die ein spritzenkörperseitiges Ende 52 der Kanüle 26 der Nadel 18 in Umfangsrichtung dicht umschließt und im zusammengesetzten Zustand der Spritze 10 mittels der Dichtungselemente 42, 44 zwischen dem Verbindungselement bzw. der Hülse 22 und der nadelseitigen Stirnseite 46 des Kopplungsabschnitts 20 gehalten ist. Dabei weist die Nadelhalterung 50 einen sich zum Kopplungsabschnitt 20 hin verjüngenden Konus 54 und einen mit dem Konus 54 nadelseitig verbundenen Kragen 56 auf. Der Kragen 56 erstreckt sich in radialer Richtung im Wesentlichen von der Kanüle 26 der Nadel 18 bis zum Innenumfang der Hülse 22 und beaufschlagt mit seiner spritzenkörperseitigen Auflagefläche 60 das Dichtungselement 42, das bevorzugt als Ringdichtung mit kreisförmigem Querschnitt ausgebildet ist. Das Dichtungselement 42 ist wiederum durch ein weiteres Dichtungs- bzw. Stützelement 44 abgestützt. Dieses Dichtungs- bzw. Stützelement 44 weist eine zum Konus 54 korrespondierende trichterartige Form auf. Bei axialer Verschiebung der Hülse 22 und der darin aufgenommenen Nadelhalterung 50 in Richtung zum Kopplungsbaschnitt 20 hin, übt der Kragen 56 in axialer Richtung wirkende Kräfte auf die Dichtungselemente 42, 44 aus. Der Konus 54 übt aufgrund seiner Geometrie auch Kräfte aus, die radiale Komponenten aufweisen, so dass die Dichtungselemente 42, 44 zwischen der Hülse 22 und dem Kopplungsabschnitt 20 vorteilhaft verspannt und zusammengepresst werden, um die gewünschte hohe Dichtigkeit der mechanischen Verbindung zwischen Nadel 18 und Spritzenkörper 14 zu erreichen.

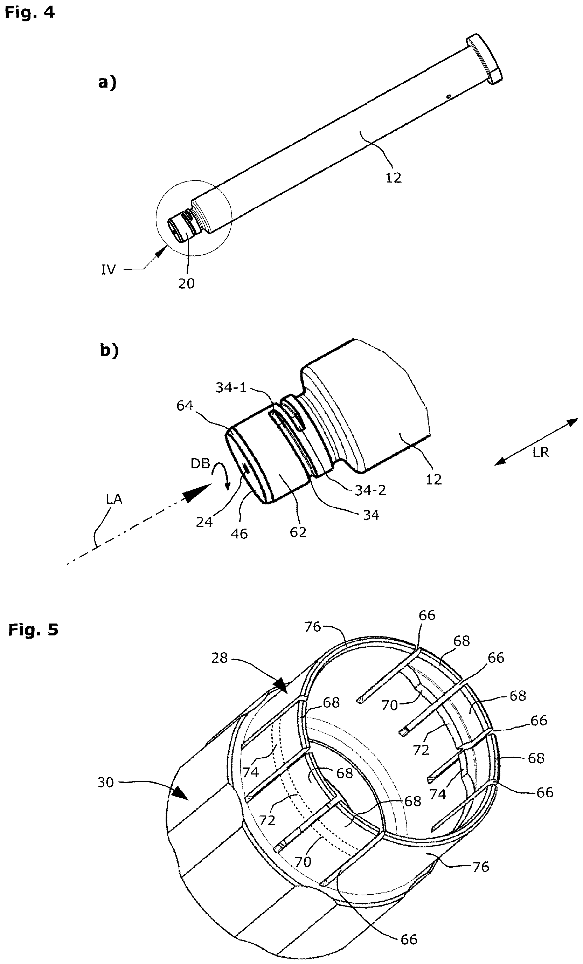

Fig. 4a zeigt den Spritzenkörper 14 in einer vereinfachten und schematischen Perspektivdarstellung mit seinem Kopplungsabschnitt 20. Fig. 4b ist eine vergrößerte Darstellung des Kopplungsabschnitts 20 entsprechend dem Bereich IV der Fig. 4a. Aus dieser Darstellung ist ersichtlich, dass die Ausnehmung 34 umlaufend um den Kopplungsabschnitt 20 ausgebildet ist und eine Steigung in axialer Richtung aufweist. Bevorzugt ist die Ausnehmung in Form eines Gewindegangs mit zwei Übergängen 34-1 und 34-2 ausgebildet. Die Übergänge 34-1 und 34-2 verbinden die Außenumfangsfläche 62 des Kopplungsabschnitts mit der Ausnehmung 34. Aus der Darstellung der Fig. 4b ist ferner ersichtlich, dass der Kopplungsabschnitt anschließend an seine nadelseitige Stirnseite 46 einen geneigten Führungsabschnitt 64 aufweist, der den Übergang zwischen der Stirnseite 46 und der Außenumfangsfläche 62 bildet. In der Stirnseite 46 ist ferner der Kanal 24 ersichtlich, der die Fluidverbindung zum Aufnahmeraum im Inneren des Spritzenkörpers 12 bildet.

Fig. 5 zeigt in schematischer perspektivischer Darstellung das Verbindungselement 22 bzw. die Hülse 22 mit dem Verbindungsabschnitt 28 und dem Stützabschnitt 30. Der Verbindungsabschnitt 28 weist entlang seines Umfangs verteilt mehrere Einschnitte oder Aussparungen 66 auf. Durch diese Einschnitte 66 werden in radialer Richtung elastisch auslenkbare Verbindungsabschnittsteile 68 gebildet werden. An den Innenumfangsflächen dieser Verbindungsabschnittsteile 68, die man auch als Verbindungszungen 68 bezeichnen kann, sind jeweilige Rastabschnittsteile 70, 72 und 74 ausgebildet, die gemeinsam einen Rastabschnitt 32 bilden. Wie aus der Darstellung der Fig. 5 ersichtlich, sind in diesem Ausführungsbeispiel zwei sich gegenüber angeordnete Rastabschnitte 32 ausgebildet, wobei der in der Figur rechte Rastabschnitt 32 sichtbar ist und der linke Rastabschnitt 32 mit seinen Rastabschnittsteilen 70, 72, 74 gestrichelt angedeutet und nicht sichtbar ist. In Umfangsrichtung liegen zwischen den beiden Rastabschnitten 32 jeweils nicht auslenkbare Verbindungsabschnittsteile 76. Im vorliegenden Beispiel erstrecken sich die Verbindungsabschnittsteile 76 und die jeweils drei Verbindungszungen 68 über etwa einen Viertelkreis in Umfangsrichtung. Selbstverständlich ist diese Anordnung der Verbindungszungen 68 und der Verbindungabschnittsteile 70 auch anders denkbar. Beispielsweise könnten auch drei Mal eine oder drei Mal zwei Verbindungszungen mit jeweils 120° Versatz angeordnet werden. Die Anzahl der Verbindungszungen und deren Anordnung verteilt entlang des Umfangs kann entsprechend den Dimensionen der Spritze und den Anforderungen an die zu erzielende Verbindung angepasst werden. Das hier dargestellte Beispiel mit zwei Mal drei Verbindungszungen 68 ist daher nicht zwingend als einschränkend hinsichtlich der Ausgestaltung der Verbindungszungen 68 und der Rastabschnitte 32 bzw. der Rastabschnittsteile 70, 72, 74 zu verstehen.

Hinsichtlich der Ausgestaltung der Rastabschnitte 32 wird auf die schematische und vereinfachte Teildarstellung der Fig. 3 hingewiesen. In dieser Darstellung ist ein Teil der Hülse 22 entsprechend dem Bereich III der Fig. 2 ohne den Kopplungsabschnitt 20 dargestellt. In beiden Beispielen weisen die Rastabschnitte 32 eine geneigt verlaufende Führungsfläche 32-1 auf. Ferner weisen beide Rastabschnitte 32 nadelseitig Auflageflächen 32-2 auf. Diese Auflageflächen 32-2 liegen an entsprechenden Seitenflächen der bevorzugt als Gewindegang ausgebildeten Ausnehmung 34 an und stützen die axial wirkenden Kräfte im zusammengesetzten Zustand der Spritze ab. Das obere Beispiel zeigt eine Auflagefläche 32-2, die im Wesentlichen orthogonal zu axialen Längsrichtung LR ausgerichtet ist. Die Auflagefläche 32-2 des unteren Beispiels ist relativ zur axialen Richtung geneigt, derart, dass der Rastabschnitt 32 die Form eines Widerhakens aufweist. Durch eine derartige Ausgestaltung des Rastabschnitts 32 wie im unteren Beispiel wird die Verbindung zwischen Verbindungselement 22 und Kopplungselement 20 noch verbessert im Verglech mit der im Wesentlichen orthogonalen Ausgestaltung (oberes Beispiel). Ein versehentliches Lösen dieser mechanischen Verbindung ist in beiden Fällen nur schwer möglich, im Falle des unteren Beispiels ist es insbesondere nur möglich unter Einwirkung von Kräften, die zum Abbrechen von Verbindungsabschnittsteilen bzw. Verbindungszungen 68 oder Rastabschnittsteilen 70, 72, 74 (Fig. 5) führen würde.

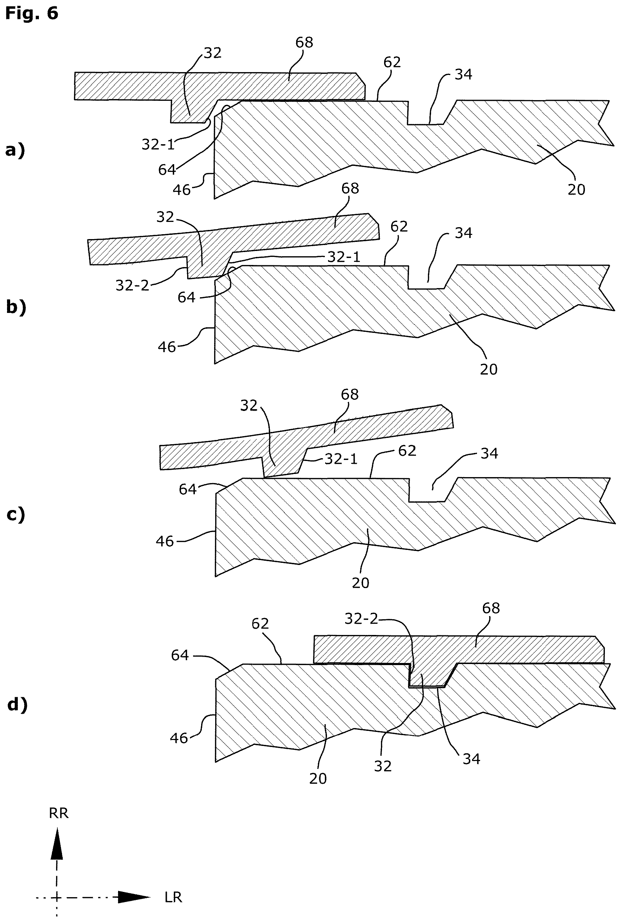

In Fig. 6 ist vereinfacht und schematisch das Herstellen der Rast- bzw. Schnappverbindung zwischen der Hülse 22 und dem Kopplungsabschnitt 20 bei einem Aufschieben der Hülse 22 in axialer Längsrichtung LR auf den Kopplungsabschnitt 20 dargestellt. Zu Beginn des Vorgangs (a) kommen die Rastabschnitte 32 mit ihren Führungsflächen 32-1 in Kontakt mit dem geneigten Führungsabschnitt 64 des Kopplungsabschnitts 20. Die geneigten Flächen der Rastabschnitte 32 und des Führungsabschnitts 64 weisen dabei eine frei wählbare Neigung auf, die derart gewählt ist, dass bei einer axialen Bewegung des Verbindungselements 22 zum Kopplungsabschnitt 20 hin, die Rastabschnitte 32 in radialer Richtung RR an dem Führungsabschnitt 64 abgewiesen werden (b). Dabei erfolgt eine radiale elastische Auslenkung der Verbindungszungen 68 nach außen (Fig. 5). Die Rastabschnitte 32 liegen dann entlang der Außenumfangsfläche des Kopplungsabschnitts 20 an (c) und rasten bzw. schnappen bei weiterem axialen Aufschieben der Hülse 22 aufgrund der wirkenden Vorspannkräfte bei der elastischen Auslenkung in die Ausnehmung 34 ein (d). Sobald die Rastabschnitte 32 in die Ausnehmungen 34 eingerastet sind, kann durch eine Drehbewegung im Uhrzeigersinn (siehe Pfeil DB in Fig. 5), die vorzugsweise etwa 1/8 bis maximale 7/8 einer vollen Drehung beträgt, insbesondere etwa 1/4 bis 1/3 einer vollen Drehung, die Hülse 22 mit dem Kopplungsabschnitt 20 dicht verbunden werden. Die mechanische bzw. formschlüssige und reibschlüssige Verbindung zwischen der Hülse 22 und dem Kopplungsabschnitt 20 erfolgt also durch eine Kombination aus einem axialen Aufschieben mit Verrastung und einer anschließenden kleinen Drehbewegung. Es ist in diesem Zusammenhang darauf hinzuweisen, dass die Rastabschnitte 32 entsprechend der Steigung des Gewindegangs der Ausnehmung 34 im Verbindungsabschnitt 28 angeordnet sind, d.h. ebenfalls eine entsprechende Steigung aufweisen, etwa in der Art eines Teil-Innengewindes. Der beschriebene Vorgang beim Zusammensetzen der fertigen Spritze 10 stellt in der End-Montage einen einfachen Fügeprozess dar, der zeitlich weniger aufwändig ist als das Vorsehen von Klebeverbindungen ggf. in Kombination mit Gewindeverbindungen, bei denen mehrere Umdrehungen von zu verbindenden Bauteilen zueinander erforderlich sind.

Es wird schließlich noch darauf hingewiesen, dass der Spritzenkörper 12 bevorzugt einstückig mit dem Kopplungsabschnitt 20 ausgebildet ist. Als Material für den Spritzenkörper 12 ist Glas bevorzugt, aber auch ein Kunststoff, der für die Laborzwecke geeignet ist, kann zum Einsatz kommen. Das Verbindungselement bzw. die Hülse 22 ist bevorzugt aus einem Kunststoff hergestellt, der vorzugsweise in einem Temperaturbereich von etwa +300°C bis -70°C eingesetzt werden kann.

Zusammenfassend wird also eine Spritze 10 nach Anspruch 1 für Laboranwendungen, wie etwa Chromatographieanwendungen, bereitgestellt, bei der das Verbindungselement (Hülse) 22 und der Kopplungsabschnitt 20 eine Schnappverbindung eingehen. Dabei ist die Schnappgeometrie (Rastabschnitte 32, Ausnehmung 34) so gestaltet, dass nach dem Einrasten eine axiale Vorspannung aufgebracht werden kann durch das Festziehen des Verbindungselements 22 mittels einer Teildrehung um die Längsachse LA. Mit dieser axialen Vorspannung können die im Verbindungselement 22 integrierten Dichtungs- bzw- Stützelemente 42, 44 vorgespannt werden, so dass eine gewünschte und für die Laboranwendungen ausreichende Dichtigkeit der Spritze 10 erreicht wird. Die hier vorgestellte Spritze eignet sich insbesondere für den Einsatz in der Hochtemperatur-Chromatographie, der Headspace-Chromatographie, in Umgebungen mit Lösemitteln (kein Klebstoff, der geschädigt werden könnte), für hochreine Anwendungen, bei denen keine organischen Stoffe (aus Klebstoffen) ausgasen dürfen, und auch für Anwendungen, bei denen eine große Zugbelastung auf die Verbindung zwischen Nadel und Spritzenkörper wirkt.

## Patentansprüche

1. Spritze, umfassend:
einen Spritzenkörper (12) mit einer axialen Innenausnehmung (16), die sich in Richtung einer Längsachse (LA) des Spritzenkörpers (12) erstreckt und in der ein Kolben (14) in Längsrichtung verschiebbar aufnehmbar oder aufgenommen ist,
und eine Nadel (18), die an einem Längsende des Spritzenkörpers (12) mit diesem verbindbar oder verbunden ist,
wobei der Spritzenkörper (12) an seinem nadelseitigen Längsende einen Kopplungsabschnitt (20) aufweist, an dem die Nadel (18) mittels eines in Längsrichtung aufschiebbaren Verbindungselements (22) befestigbar oder befestigt ist,
wobei am Kopplungsabschnitt (20) wenigstens eine radial nach innen ausgebildete Ausnehmung (34) vorgesehen ist, die mit wenigstens einem korrespondierenden am Verbindungselement (22) vorgesehenen Rastabschnitt (32) in Eingriff bringbar ist oder steht,
wobei das Verbindungselement (22) als Hülse ausgebildet ist, die einen spritzenkörperseitigen Verbindungsabschnitt (28) und einen nadelseitigen Stützabschnitt (30) umfasst, wobei der wenigstens eine Rastabschnitt (32) am Verbindungsabschnitt (28) angeordnet ist,
wobei am Verbindungsabschnitt (28) mehrere in Umfangsrichtung verteilte Rastabschnitte (32) vorgesehen sind, die sich entlang des Innenumfangs der Hülse (22) erstrecken und radial nach innen weisen, und
dass der Verbindungsabschnitt (28) im Bereich der Rastabschnitte (32) in Längsrichtung verlaufende Einschnitte (66) aufweist, die am spritzenkörperseitigen Ende der Hülse (22) beginnen und in Richtung zum nadelseitigen Ende der Hülse (22) weisen, derart, dass die Rastabschnitte (32) mehrere Rastabschnittsteile (70, 72, 74) aufweisen, die an entsprechenden Verbindungszungen (68) angeordnet sind, wobei die Verbindungszungen (68) in radialer Richtung (RR) auslenkbar sind, wobei in Umfangsrichtung zwischen den verteilt angeordneten Rastabschnitten (32) nicht auslenkbare Verbindungsabschnittsteile (76) des Verbindungsabschnitts (28) liegen,
**dadurch gekennzeichnet, dass** die Spritze ferner eine Spanneinrichtung aufweist, mittels welcher eine axiale Vorspannung zwischen Kopplungsabschnitt (20) und Verbindungselement (22) herstellbar oder hergestellt ist, die auf Dichtungselemente (42, 44) wirkt, welche zwischen dem Kopplungsabschnitt (20) und dem Verbindungselement (22) aufgenommen sind,
wobei die Spanneinrichtung die wenigstens eine Ausnehmung (34) am Kopplungsabschnitt (20) umfasst, und wobei sich die Ausnehmung (34) entlang des Umfangs des Kopplungsabschnitts (20) erstreckt und in Form eines Gewindegangs ausgebildet ist.

2. Spritze nach Anspruch 1, **dadurch gekennzeichnet, dass** der Gewindegang (34) derart ausgebildet ist, dass er in beide Drehrichtungen einen Übergang (34-1, 34-2) zu einer Außenumfangsfläche (62) des Kopplungsabschnitts (20) aufweist, und dass vorzugsweise maximal eine Umdrehung des Verbindungselements (22) um den Kopplungsabschnitt (34) möglich ist, vorzugsweise eine Teildrehung um etwa 1/8 bis 7/8 des Umfangs des Kopplungsabschnitts (20).

3. Spritze nach einem der vorhergehenden Ansprüche, **dadurch gekennzeichnet, dass** die Hülse (22) an ihrem Stützabschnitt (30) eine radial nach innen weisende Begrenzungswandung (36) aufweist, durch deren Zentrum (38) die Nadel (18) geführt ist, wobei im zusammengesetzten Zustand der Spritze die Begrenzungswandung (36) mit ihrer dem Spritzenkörper (12) zugewandten Innenfläche (40) eine axiale Kraft auf die an einer axialen Stirnfläche (46) des Kopplungsabschnitts (20) anliegenden Dichtungselemente (42, 44) ausübt.

4. Spritze nach Anspruch 3, **dadurch gekennzeichnet, dass** der Stützabschnitt (30) ferner einen radial nach innen weisenden Stabilisierungsring (48) aufweist, der im zusammengesetzten Zustand der Spritze (10) an der Stirnfläche (46) des Kopplungsabschnitts (20) anliegt, wobei vorzugsweise der Stabilisierungsring (48) einstückig mit der Hülse (22) ausgebildet ist.

5. Spritze nach einem der vorhergehenden Ansprüche, **dadurch gekennzeichnet, dass** die Dichtungselemente (42, 44) und die Nadel (18) im Verbindungselement (22) bzw. in der Hülse (22) aufgenommen, vorzugsweise befestigt sind, so dass sie als gemeinsames Bauteil mit dem Kopplungsabschnitt (20) des Spritzenkörpers (12) verbindbar oder verbunden sind.

6. Spritze nach einem der vorhergehenden Ansprüche, **dadurch gekennzeichnet, dass** der Spritzenkörper (12) und der Kopplungsabschnitt (20) einstückig ausgebildet sind, vorzugsweise aus Glas.

7. Spritze nach einem der vorhergehenden Ansprüche, **dadurch gekennzeichnet, dass** das Verbindungselement (22) bzw. die Hülse (22) aus einem Kunststoff gebildet ist.

8. Spritze nach einem der vorhergehenden Ansprüche, ferner umfassend eine im Verbindungselement (22) bzw. der Hülse (22) aufgenommene Nadelhalterung (50), welche ein spritzenkörperseitiges Ende (52) einer Kanüle (26) der Nadel (18) in Umfangsrichtung dicht umschließt und im zusammengesetzten Zustand der Spritze (10) mittels Dichtungselementen (42, 44) zwischen dem Verbindungselement (22) bzw. der Hülse (22) und der nadelseitigen Stirnfläche (46) des Kopplungsabschnitts (30) gehalten ist.

## Claims

1. A syringe comprising:
a syringe body (12) having an axial inner cavity (16), which extends in the direction of a longitudinal axis (LA) of the syringe body (12) and in which a plunger (14) can be or is accommodated in such a way that the plunger can be moved in the longitudinal direction, and
a needle (18), which can be or is connected to the syringe body (12) at a longitudinal end of the syringe body (12), wherein
the syringe body (12) has a coupling segment (20) at its needle-side longitudinal end, to which the needle (18) can be or is fastened by means of a connecting element (22) that can be slid on along the longitudinal direction,
at least one radially inwardly formed cavity (34) is provided on the coupling segment (20), which can be brought into engagement or is in engagement with at least one corresponding locking segment (32) provided on the connecting element (22),
wherein the connecting element (22) is formed as a hull, which comprises a syringe body-side connecting segment (28) and a needle-side supporting segment (30), wherein the at least one locking segment (32) is arranged on the connecting segment (28),
wherein on the connecting segment (28) several locking segments (32) are provided distributed in a circumferential direction, the locking segments (32) extending along the inner circumference of the hull (22) and point radially inwards, and
that the connecting segment (28) comprises in the region of the locking segments (32) grooves (66) that extend in the longitudinal direction and that begin at a syringe body-side end of the hull (22) and point into the direction of the needle-side end of the hull (22) such that the locking segments (32) comprise several locking segment parts (70, 72, 74), which are arranged on corresponding connecting segment parts (68), wherein the connecting segment parts (68) can be deflected in a radial direction (RR), wherein non-deflectable connecting segment parts (76) of the connecting segment (28) lie in circumferential direction respectively between the distributed arranged locking segments (32),
**characterized in that** the syringe further comprises tensioning means, by means of which an axial pretension can be or is produced between the coupling segment (20) and the connecting element (22), the pretension acting on sealing elements (42, 44), which are accommodated between the coupling segment (20) and the connecting segment (22),
wherein the tensioning means comprises the at least one cavity (34) on the coupling segment (20), wherein the cavity (34) extends along the circumference of the coupling segment (20) and is formed as a thread.

2. Syringe according to claim 1, **characterized in that** the thread is formed such that it has a transition (34-1, 34-2) to an outer circumferential surface (62) of the coupling segment (20) in both turning directions, and that preferably maximally one turn of the connecting element (22) with respect to the coupling segment (34) is possible, preferably a partial turn around about 1/8 to 7/8 of the circumference of the coupling segment (20).

3. Syringe according to one of the preceding claims, **characterized in that** the hull (22) comprises on its supporting segment (30) a boundary wall (36) that points radially inwards, through a center of which (38) the needle (18) passes, wherein in an assembled state of the syringe the boundary wall (36) exerts with its inner surface (40) that faces the syringe body (12) an axial force onto the sealing elements (42, 44) that lie against an axial front surface (46) of the coupling surface (20).

4. Syringe according to claim 3, **characterized in that** the supporting segment (30) comprises further a stabilizing ring (48) that points radially inwards, which lies in the assembled state of the syringe (10) against the front surface (46) of the coupling segment (20), wherein the stabilizing ring (48) is preferably formed integrally with the hull (22).

5. Syringe according to one of the preceding claims, **characterized in that** the sealing elements (42, 44) and the needle (18) are accommodated, preferably fastened, in the connecting element (22) or in the hull (22) such that they can be or are connected as a combined element to the coupling segment (20) of the syringe body (12).

6. Syringe according to one of the preceding claims, **characterized in that** the syringe body (12) and the coupling segments (20) are integrally formed, preferably of glass.

7. Syringe according to one of the preceding claims, **characterized in that** the connecting element (22) or the hull (22) are formed of synthetic materials.

8. Syringe according to one of the preceding claims, further comprising a needle holder (50) accommodated in the connecting element (22) or the hull (22), which tightly encompasses a syringe body-side end (52) of a cannula (26) of the needle (18) in circumferential direction, and which is held in the assembled state of the syringe (10) by means of sealing elements (42, 44) between the connecting element (22) or the hull (22) and the needle-side front surface (46) of the coupling segment (30).

## Revendications

1. Seringue, comprenant :
un corps de seringue (12) comprenant un évidement intérieur axial (16) qui s'étend dans la direction d'un axe longitudinal (LA) du corps de seringue (12) et dans lequel un piston (14) est apte à être logé ou est logé de manière coulissante dans la direction longitudinale,
et une aiguille (18) qui, à une extrémité longitudinale du corps de seringue (12), est apte à être reliée ou est reliée à celui-ci,
dans laquelle le corps de seringue (12) comporte à son extrémité longitudinale côté aiguille une section de couplage (20) à laquelle l'aiguille (18) est apte à être fixée ou est fixée au moyen d'un élément de liaison (22) apte à être enfilé dans la direction longitudinale,
dans laquelle il est prévu sur la section de couplage (20) au moins un évidement (34) formé radialement vers l'intérieur, qui est apte à être mis en prise ou est en prise avec au moins une section d'encliquetage (32) correspondante prévue sur l'élément de liaison (22),
dans laquelle l'élément de liaison (22) est conformé en un manchon qui comprend une section de liaison côté corps de seringue (28) et une section d'appui côté aiguille (30), dans laquelle la au moins une section d'encliquetage (32) est disposée sur la section de liaison (28),
dans laquelle il est prévu sur la section de liaison (28) plusieurs sections d'encliquetage (32), réparties dans la direction circonférentielle, qui s'étendent le long de la circonférence intérieure du manchon (22) et sont dirigées radialement vers l'intérieur, et
que la section de liaison (28) comporte dans la zone des sections d'encliquetage (32) des encoches (66), s'étendant dans la direction longitudinale, qui commencent à l'extrémité côté corps de seringue du manchon (22) et sont dirigées en direction de l'extrémité côté aiguille du manchon (22), de telle sorte que les sections d'encliquetage (32) comportent plusieurs parties de sections d'encliquetage (70, 72, 74) qui sont disposées sur des languettes de liaison (68) correspondantes, dans laquelle les languettes de liaison (68) sont aptes à être déviées dans la direction radiale (RR), dans laquelle des parties de sections de liaison (76) de la section de liaison (28) non aptes à être déviées sont situées dans la direction circonférentielle entre les sections d'encliquetage (32) réparties,
**caractérisée en ce que** la seringue comporte en outre un dispositif de serrage au moyen duquel une précontrainte axiale est apte à être réalisée ou est réalisée entre la section de couplage (20) et l'élément de liaison (22), qui agit sur des éléments d'étanchéité (42, 44) qui sont logés entre la section de couplage (20) et l'élément de liaison (22),
dans laquelle le dispositif de serrage comprend le au moins un évidement (34) sur la section de couplage (20), et dans laquelle l'évidement (34) s'étend le long de la circonférence de la section de couplage (20) et est réalisé en forme de pas de vis.

2. Seringue selon la revendication 1, **caractérisée en ce que** le pas de vis (34) est formé de telle sorte qu'il comporte dans les deux directions de rotation une transition (34-1, 34-2) avec une surface circonférentielle extérieure (62) de la section de couplage (20), et que de préférence au maximum un tour de l'élément de liaison (22) autour de la section de couplage (34) est possible, de préférence une rotation partielle d'environ 1/8 à 7/8 de la circonférence de la section de couplage (20).

3. Seringue selon l'une des revendications précédentes, **caractérisée en ce que** le manchon (22) comporte sur sa section d'appui (30) une paroi de limitation (36) dirigée radialement vers l'intérieur, grâce au centre (38) de laquelle l'aiguille (18) est guidée, dans laquelle à l'état assemblé de la seringue, la paroi de limitation (36) exerce avec sa surface intérieure (40) tournée vers le corps de seringue (12) une force axiale sur les éléments d'étanchéité (42, 44) appliqués contre une surface frontale axiale (46) de la section de couplage (20).

4. Seringue selon la revendication 3, **caractérisée en ce que** la section d'appui (30) comporte en outre une bague de stabilisation (48) dirigée radialement vers l'intérieur, qui, dans l'état assemblé de la seringue (10), est appliquée contre la surface frontale (46) de la section de couplage (20), dans laquelle de préférence la bague de stabilisation (48) est formée d'un seul tenant avec le manchon (22).

5. Seringue selon l'une des revendications précédentes, **caractérisée en ce que** les éléments d'étanchéité (42, 44) et l'aiguille (18) sont logés, de préférence fixés, dans l'élément de liaison (22) ou manchon (22), de sorte qu'ils soient aptes à être reliés ou sont reliés, en tant que composant commun, à la section de couplage (20) du corps de seringue (12).

6. Seringue selon l'une des revendications précédentes, **caractérisée en ce que** le corps de seringue (12) et la section de couplage (20) sont formés d'un seul tenant, de préférence en verre.

7. Seringue selon l'une des revendications précédentes, **caractérisée en ce que** l'élément de liaison (22) ou manchon (22) est formé en une matière plastique.

8. Seringue selon l'une des revendications précédentes, comprenant en outre une fixation d'aiguille (50) logée dans l'élément de liaison (22) ou manchon (22), qui entoure de manière étanche une extrémité côté corps de seringue (52) d'une canule (26) de l'aiguille (18) dans la direction circonférentielle, et, dans l'état assemblé de la seringue (10), est maintenue au moyen d'éléments d'étanchéité (42, 44) entre l'élément de liaison (22) ou manchon (22) et la surface frontale côté aiguille (46) de la section de couplage (20).
